# EUROPÄISCHE PATENTANMELDUNG

(11) **EP 4 512 434 A1**
(43) Veröffentlichungstag der Anmeldung: **26.02.2025**
(21) Anmeldenummer: 23193040.5
(22) Anmeldetag: 23.08.2023
(51) Int. Cl.: A61L 9/12, B65D 5/72

(54) **GEHÄUSE UND VORRICHTUNG ZUR ABGABE EINES FLÜCHTIGEN STOFFES, INSBESONDERE DUFTSTOFFS ODER INSEKTIZIDEN WIRKSTOFFS, AN DIE UMGEBUNGSLUFT**

(71) Anmelder: Swif GmbH South West International Fragrance, 67661 Kaiserslautern (DE)
(72) Erfinder: KARG, Joern, 67661 Kaiserslautern (DE)
(74) Vertreter: Prescher, Gordian

(57) **Zusammenfassung**

Die vorliegende Erfindung betrifft eine Vorrichtung (100) zur Abgabe eines flüchtigen Stoffs, insbesondere eines Duftstoffs oder eines insektiziden Wirkstoffes, an die Umgebungsluft,
mit einem Gehäuse (1) aus einem ersten Material, welches einen pflanzlichen Faserstoff, insbesondere ein Papier, einen Karton oder eine Pappe, aufweist und
mit einer innerhalb des Gehäuses (1) angeordneten Einlage (30) aus einem zweiten Material, die mit dem flüchtigen Stoff versehen ist,
wobei das Gehäuse (1) ein erstes Gehäuseteil (10) und ein zweites Gehäuseteil (20) umfasst, wobei das erste Gehäuseteil (10) und/oder das zweite Gehäuseteil (20) eine Aufnahme für die Einlage (30) umfasst,
wobei das erste Gehäuseteil (10) eine oder mehrere Durchgangsöffnungen (12) aufweist, durch welche der flüchtige Stoff in die Umgebungsluft abgegeben werden kann, und das zweite Gehäuseteil (20) aus einer Schließstellung, in welcher es die Durchgangsöffnungen (12) des ersten Gehäuseteils (10) verdeckt, in eine Offenstellung verschiebbar ist, in welcher die Durchgangsöffnungen (12) des ersten Gehäuseteils (10) zur Abgabe des flüchtigen Stoffs freigegeben sind,
wobei an dem ersten und an dem zweiten Gehäuseteil (10, 20) korrespondierende Rastelemente (14, 24) angeordnet ist, die in einem Rastzustand ein vollständiges Trennen der beiden Gehäuseteile (10, 20) voneinander verhindern,
wobei das erste Gehäuseteil (10) und/oder das zweite Gehäuseteil (20) einen durch mindestens eine Falzlinie (11) begrenzten Betätigungsbereich (16) aufweist, der dazu eingerichtet ist, bei einer Druckeinwirkung durch einen Benutzer der Vorrichtung die korrespondierenden Rastelemente (14, 24) aus dem Rastzustand in einem Freigabezustand zu versetzen, in welchem die beiden Gehäuseteile (10, 20) vollständig voneinander trennbar sind. Ferner betrifft die Erfindung ein Gehäuse (1) für eine derartige Vorrichtung (100).

## Beschreibung

### Stand der Technik

Die Erfindung betrifft ein Gehäuse aus einem ersten Material, welches einen pflanzlichen Faserstoff, insbesondere ein Papier, einen Karton oder eine Pappe, aufweist, wobei das Gehäuse ein erstes Gehäuseteil und ein zweites Gehäuseteil umfasst, wobei an dem ersten und an dem zweiten Gehäuseteil korrespondierende Rastelemente angeordnet sind, die in einem Rastzustand ein vollständiges Trennen der beiden Gehäuseteile voneinander verhindern. Ferner betrifft die Erfindung eine Vorrichtung zur Abgabe eines flüchtigen Stoffs, insbesondere eines Duftstoffs oder eines insektiziden Wirkstoffes, an die Umgebungsluft mit einem derartigen Gehäuse und einer innerhalb des Gehäuses angeordneten Einlage aus einem zweiten Material, die mit dem flüchtigen Stoff versehen ist, wobei das Gehäuse ein erstes Gehäuseteil und ein zweites Gehäuseteil aufweist, wobei das erste Gehäuseteil und/oder das zweite Gehäuseteil eine Aufnahme für die Einlage umfasst, wobei das erste Gehäuseteil eine oder mehrere Durchgangsöffnungen aufweist, durch welche der flüchtige Stoff in die Umgebungsluft abgegeben werden kann, und das zweite Gehäuseteil aus einer Schließstellung, in welcher es die Durchgangsöffnungen des ersten Gehäuseteils verdeckt, in eine Offenstellung verschiebbar ist, in welcher die Durchgangsöffnungen des ersten Gehäuseteils zur Abgabe des flüchtigen Stoffs freigegeben sind.

Derartige Vorrichtungen sind beispielsweise aus US 4 279 373 A und DE 10 2020 120 951 A1 bekannt und umfassen ein Gehäuse mit zwei Gehäuseteilen. Innerhalb des Gehäuses ist eine als Einlage ausgebildete Abgabeeinrichtung angeordnet, die während des Gebrauchs der Vorrichtung einen flüchtigen Stoff, z. B. einen Duftstoff, abgeben kann. Um den Zugang zu der Abgabeeinrichtung, beispielswiese für kleine Kinder, zu erschweren, sind an den Gehäuseteilen Rastelemente vorgesehen, die ein vollständiges Trennen der beiden Gehäuseteile voneinander verhindern. Bei diesen Vorrichtungen sind die Rastelemente so ausgelegt, dass sich die beiden Gehäuseteile nur schwer zerstörungsfrei voneinander lösen lassen. Hierdurch wird das Wiederverwenden des Gehäuses nach dem Verbrauch der Abgabeeinrichtung erschwert.

### Offenbarung der Erfindung

Vor diesem Hintergrund stellt sich die Aufgabe, die Wiederverwendbarkeit des derartigen Gehäuses zu erhöhen.

Die Aufgabe wird gelöst durch ein Gehäuse aus einem ersten Material, welches einen pflanzlichen Faserstoff, insbesondere ein Papier, einen Karton oder eine Pappe, aufweist, wobei das Gehäuse ein erstes Gehäuseteil und ein zweites Gehäuseteil umfasst, wobei an dem ersten und an dem zweiten Gehäuseteil korrespondierende Rastelemente angeordnet sind, die in einem Rastzustand ein vollständiges Trennen der beiden Gehäuseteile voneinander verhindern, wobei das erste Gehäuseteil und/oder das zweite Gehäuseteil einen durch mindestens eine Falzlinie begrenzten Betätigungsbereich aufweist, der dazu eingerichtet ist, bei einer Druckeinwirkung durch einen Benutzer der Vorrichtung die korrespondierenden Rastelemente aus dem Rastzustand in einem Freigabezustand zu versetzen, in welchem die beiden Gehäuseteile vollständig voneinander trennbar sind.

Das Gehäuse kann als Verpackung für Produkte Verwendung, bevorzugt Haushaltsprodukte, insbesondere bevorzugt Haushaltsprodukte, welche vor schützenswerten Personengruppen unzugänglich aufbewahrt werden müssen, finden, beispielsweise Reinigungsmittel, Waschparfüm, insbesondere in Form von Pulver, Perlen, Caps oder Pods, oder Waschmitteln oder Lebensmitteln. Darüber hinaus kann das Produkt auch zur Insektenbekämpfung dienen, wobei einerseits eine Insektenfalle, insbesondere zur Anziehung und dem gleichzeitigen Fangen von Insekten, und andererseits eine abschreckende Wirkung gegenüber Insekten denkbar ist.

Das erfindungsgemäße Gehäuse besteht aus einem ersten Material. Dieses erste Material weist einen pflanzlichen Faserstoff auf, beispielsweise ein Papier, einen Karton oder eine Pappe, und ist daher nach dem Gebrauch ohne großen Aufwand zu entsorgen bzw. zu recyclen. Weiterhin kann das Papier mehrschichtig sein, wobei bevorzugt eine Schicht als Barriereschicht ausgestaltet ist und eine Barrierefunktion aufweist. Die Barriereschicht ist bevorzugt die innerste oder die äußerste Schicht des mehrschichtigen Papieres. Das erste Gehäuseteil und das zweite Gehäuseteil umfassen erfindungsgemäß korrespondierende Rastelemente, wobei diese beim Einschieben des ersten Gehäuseteils in das zweite Gehäuseteil verrasten. Das vollständige Trennen des ersten Gehäuseteils vom zweiten Gehäuseteil kann somit verhindert werden, sodass schützenswerte Personengruppen, insbesondere Kinder, keinen Zugang zu den Produkten innerhalb des Gehäuses erlangen können. Durch das erfindungsgemäße Vorsehen mindestens einer Falzlinie wird ein Betätigungsbereich optisch markiert und gleichzeitig ein Eindrücken des Betätigungsbereichs durch einen Benutzer ermöglicht. Durch das Eindrücken des Betätigungsbereichs können die Rastelemente aus ihrem Rastzustand in einen Freigabezustand versetzt werden, in welchem die korrespondierenden Rastelemente voneinander lösbar sind. Der Freigabezustand ermöglicht die Trennung des ersten Gehäuseteils vom zweiten Gehäuseteil. Bei voneinander getrennten Gehäuseteilen können ein oder mehrere Produkte entnommen und das Gehäuse als Verpackung weiter- bzw. wiederverwendet werden.

Die Aufgabe wird ferner gelöst durch eine Vorrichtung zur Abgabe eines flüchtigen Stoffs, insbesondere eines Duftstoffs oder eines insektiziden Wirkstoffes, an die Umgebungsluft, mit einem Gehäuse aus einem ersten Material, welches einen pflanzlichen Faserstoff, insbesondere ein Papier, einen Karton oder eine Pappe, aufweist und mit einer innerhalb des Gehäuses angeordneten Einlage aus einem zweiten Material, die mit dem flüchtigen Stoff versehen ist, wobei das Gehäuse ein erstes Gehäuseteil und ein zweites Gehäuseteil umfasst, wobei das erste Gehäuseteil und/oder das zweite Gehäuseteil eine Aufnahme für die Einlage umfasst, wobei das erste Gehäuseteil eine oder mehrere Durchgangsöffnungen aufweist, durch welche der flüchtige Stoff in die Umgebungsluft abgegeben werden kann, und das zweite Gehäuseteil aus einer Schließstellung, in welcher es die Durchgangsöffnungen des ersten Gehäuseteils verdeckt, in eine Offenstellung verschiebbar ist, in welcher die Durchgangsöffnungen des ersten Gehäuseteils zur Abgabe des flüchtigen Stoffs freigegeben sind, wobei an dem ersten und an dem zweiten Gehäuseteil korrespondierende Rastelemente angeordnet ist, die in einem Rastzustand ein vollständiges Trennen der beiden Gehäuseteile voneinander verhindern, wobei das erste Gehäuseteil und/oder das zweite Gehäuseteil einen durch mindestens eine Falzlinie begrenzten Betätigungsbereich aufweist, der dazu eingerichtet ist, bei einer Druckeinwirkung durch einen Benutzer der Vorrichtung die korrespondierenden Rastelemente aus dem Rastzustand in einem Freigabezustand zu versetzen, in welchem die beiden Gehäuseteile vollständig voneinander trennbar sind.

Derartige Vorrichtungen können auch als Raumlufterfrischer oder Insektenvernichter bezeichnet werden. Der Insektenvernichter kann die Insekten anlocken, bzw. töten oder absto-ßen. Das Anlocken der Insekten kann die Insekten innerhalb der sogenannten Insektenfalle fangen, wobei die Insekten in dieser entweder an einem dafür vorgesehenen Klebstoff haften bleiben und verenden oder durch den abgegebenen Stoff an sich verenden. Hierbei ist es auch denkbar, dass die Insekten den Stoff einnehmen, insbesondere essen, und durch diesen sterben. Alternativ kann die Insektenfalle auch einen Stoff aufweisen, welchen die Insekten an sich oder zu sich nehmen, teilweise oder ganz, und zu den Nestern transportieren, wobei die anderen Insekten auch an diesem Stoff verenden.

Die erfindungsgemäße Vorrichtung umfasst ein Gehäuse aus einem ersten Material. Dieses erste Material weist einen pflanzlichen Faserstoff auf, beispielsweise ein Papier, einen Karton oder eine Pappe, und ist daher nach dem Gebrauch ohne großen Aufwand zu entsorgen bzw. zu recyclen. Das Papier kann bevorzugt mehrschichtig sein, wobei insbesondere bevorzugt eine Schicht als Barriereschicht ausgestaltet ist und eine Barrierefunktion aufweist. Die Barriereschicht ist bevorzugt die innerste oder die äußerste Schicht des mehrschichtigen Papieres und kann beispielsweise aus Polyethylen (PE) oder aus orientiertem Polypropylen (z.B. (B)OPP) bestehen. Ein weiterer Bestandteil der Vorrichtung ist eine Einlage aus einem zweiten Material, welches sich von dem ersten Material unterscheidet. Somit können das erste und zweite Material spezifisch für die jeweilige Funktion ausgewählt werden. Denn während das erste Material des Gehäuses vor der Benutzung der Vorrichtung eine Barrierefunktion für den flüchtigen Stoff bilden muss, sollte das zweite Material möglichst gute für die Abgabe des flüchtigen Stoffs relevante Eigenschaften haben. Das zweite Material ist bevorzugt ein pflanzlicher Faserstoff, insbesondere ein Papier, ein Karton oder eine Pappe. Alternativ kann es sich bei dem zweiten Material auch um eine Kunstfaser oder Saccharose oder Stärke oder ein mineralisches Material handeln, welche besonders geeignet ist zur Aufnahme und Abgabe von flüchtigen Stoffen an die Umgebungsluft. Das zweite Material ist mit dem flüchtigen Stoff versehen, insbesondere imprägniert, so dass dieser aus dem Gehäuse an die Umgebungsluft abgegeben werden kann. Die Einlage kann auch mehrschichtig sein, wobei die Materialien der jeweiligen Schichten sich bevorzugt unterscheiden. Die jeweiligen Schichten werden insbesondere zusammengenäht, genagelt, getuckert, gepresst, geklebt oder auch zusammengebunden. Eine Schicht der Einlage weist vorteilhafterweise ein saugfähiges bzw. für die flüchtigen Stoffe aufnahmefähiges Material auf. Die Einlage kann ein Lockstoff für Insekten, insbesondere mit einem Pheromon getränkt werden, um Insekten anzulocken. Bevorzugt weist das erste Gehäuseteil oder das zweite Gehäuseteil auf einer oder mehrerer der Einlage zugewandten Flächen eine Klebefolie auf, wobei die Insekten hier kleben bleiben. Alternativ kann die Einlage auch einen für Insekten abstoßenden Stoff aufweisen, wobei die Einlage durch das Gehäuse geschützt wird und somit in vorteilhafterweise ein direkter Kontakt der Einlage mit beispielsweise Anziehsachen vermieden werden kann. Auch die Einlage der Vorrichtung ist nach dem Gebrauch ohne großen Aufwand zu entsorgen bzw. zu recyclen. Es ist möglich, das Gehäuse und die Einlage gemeinsam, d.h. ohne sie trennen zu müssen, zu entsorgen, z.B. im Papiermüll oder Verpackungsmüll ("gelber Sack"). Zudem ist das Gehäuse zweigeteilt ausgestaltet, sodass die Vorrichtung ein erstes Gehäuseteil und ein zweites Gehäuseteil umfasst. Zur Abgabe eines Duftstoffes oder eines insektiziden Wirkstoffes umfasst das erste und/oder das zweite Gehäuseteil eine Aufnahme für eine Einlage. Das erste Gehäuseteil weist erfindungsgemäß eine oder mehrere Durchgangsöffnungen auf, wodurch der flüchtige Stoff in der Offenstellung an die Umgebungsluft abgegeben wird. Um den flüchtigen Stoff innerhalb des Gehäuses zu halten, werden die Durchgangsöffnungen erfindungsgemäß, sofern vom Nutzer gewünscht, in eine Schließstellung gebracht. In dieser Schließstellung werden die Durchgangsöffnungen des ersten Gehäuseteils durch das zweite Gehäuseteil vollständig abgedeckt. Vorteilhafterweise kann somit die Anwendung der jeweiligen Nutzung der Vorrichtung unterbrochen werden. Weiterhin ist es denkbar, dass durch die Reibung zwischen dem ersten und dem zweiten Gehäuseteil eine stufenlose Verstellbarkeit möglich ist. Vorteilhafterweise kann somit der Grad der Öffnung der Durchgangsöffnungen eingestellt werden, sodass vorteilhafterweise eine Dosierbarkeit gegeben ist. Das erste Gehäuseteil und das zweite Gehäuseteil umfassen erfindungsgemäß korrespondierende Rastelemente, wobei diese beim Einschieben des ersten Gehäuseteils in das zweite Gehäuseteil verrasten. Das vollständige Trennen des ersten Gehäuseteils vom zweiten Gehäuseteil kann somit verhindert werden, sodass schützenswerte Personengruppen, insbesondere Kinder, keinen Zugang zur Einlage innerhalb des Gehäuses erlangen können. Durch das erfindungsgemäße Vorsehen mindestens einer Falzlinie wird ein Betätigungsbereich optisch markiert und gleichzeitig ein Eindrücken des Betätigungsbereichs durch einen Benutzer ermöglicht. Durch das Eindrücken des Betätigungsbereichs können die Rastelemente aus ihrem Rastzustand in einen Freigabezustand versetzt werden, in welchem die korrespondierenden Rastelemente voneinander lösbar sind. Der Freigabezustand ermöglicht die Trennung des ersten Gehäuseteils vom zweiten Gehäuseteil. Bei voneinander getrennten Gehäuseteilen kann eine Einlage, die den flüchtigen Stoff, mit dem sie versehen war, im Wesentlichen abgegeben hat, ("verbrauchte" Einlage) entnommen und gegen eine "frische" Einlage ausgetauscht werden. Insofern kann durch die erfindungsgemäße Ausgestaltung die Wiederverwendbarkeit des Gehäuses verbessert werden.

Das erste Material kann mehrere Barriereschichten aufweisen. Beispielsweise kann vorgesehen sein, dass die Barriereschichten unterschiedliche Permeabilitäten für unterschiedliche flüchtige Stoffe aufweisen. Auf diese Weise kann ein Gehäuse erhalten werden, welches eine Barrierefunktion für mehrere flüchtige Stoffe aufweist, beispielsweise für mehrere Stoffe einer Stoffmischung, z.B. einer Duftstoffmischung.

Nachfolgende sollen vorteilhafte Ausgestaltungen des erfindungsgemäßen Gehäuses erläutert werden, die wahlweise auch bei der erfindungsgemäßen Vorrichtung zur Abgabe eines flüchtigen Stoffs Anwendung finden können.

Bevorzugt ist der Betätigungsbereich dazu eingerichtet, bei der Druckeinwirkung durch den Benutzer sich zumindest eines der korrespondierenden Rastelemente verformt und/oder in Richtung eines Innenraums des Gehäuseteils bewegt wird und dadurch den Freigabezustand herstellt.

Gemäß einer vorteilhaften Ausgestaltung ist vorgesehen, dass die mindestens eine Falzlinie in dem ersten und/oder zweiten Gehäuseteil als Falzung oder Nutung oder Rillung oder Perforation ausgebildet ist. Derartig ausgebildete Falzlinien können durch ein maschinell durchführbares Verfahren ausgebildet werden. Beim Falzen wird das Material des jeweiligen Gehäuseteile entlang der Falzlinie gefaltet. Beim Nuten wird ein Stück des Materials des jeweiligen Gehäuseteils herausgeschnitten und somit eine Nut entlang der Falzlinie erzeugt. Beim Rillen wird das Material des jeweiligen Gehäuseteils eingedrückt, um die Falzlinie bereitzustellen. Beim Perforieren werden entlang der Falzlinie mehrere Löcher, insbesondere in regelmäßigen Abständen, eingebracht.

Gemäß einer vorteilhaften Ausgestaltung ist vorgesehen, dass die an einem ersten und einem zweiten Gehäuseteil vorhandenen Rastelemente aus dem jeweiligen Material des ersten oder des zweiten Gehäuseteils bestehen, wobei das Rastelement ein an dem jeweiligen ersten oder zweiten Gehäuseteil umlegbarer Abschnitt ist. Beispielsweise können die Rastelemente als umgefalzte Abschnitte des Materials des ersten bzw. zweiten Gehäuseteils ausgebildet sein. Das erste Gehäuseteil und das zweite Gehäuseteil werden bevorzugt aus einem Zuschnitt aufgebaut, sodass der Zuschnitt bereits die Rastelemente aufweist. Zur Anwendung des Rastelementes kann der am Zuschnitt vorhandene umlegbare Abschnitt nach innen geknickt werden und mit dem korrespondieren Rastelement den Rastzustand erzeugen.

Gemäß einer vorteilhaften Ausgestaltung ist vorgesehen, dass der Betätigungsbereich durch mehrere Falzlinien begrenzt ist. Insbesondere können zwei, drei, vier oder fünf Falzlinien vorgesehen sein, um den Betätigungsbereich zu begrenzen.

Gemäß einer vorteilhaften Ausgestaltung ist vorgesehen, dass der Betätigungsbereich durch zwei parallel verlaufend angeordnete Falzlinien begrenzt ist. In einer alternativen Ausgestaltungsform können die Falzlinien aufeinander zulaufend oder voneinander weglaufend ausgeführt sein. Die Falzlinien können einen oder mehrere Knicke im Verlauf aufweisen, wobei die Falzlinien vor oder nach einem Knickpunkt parallel verlaufen können. Die Falzlinien sind bevorzugt sternenförmig angeordnet, wobei die Knicke in den Verläufen derart zueinander angeordnet sind, dass sich ungefähr eine Sternenform ergibt.

Gemäß einer vorteilhaften Ausgestaltung ist vorgesehen, dass das erste Gehäuseteil und/oder das zweite Gehäuseteil mindestens einen Betätigungsbereich aufweist, wobei dieser mittig auf einer Seitenfläche des Gehäuseteils angeordnet ist. Der Betätigungsbereich wird optisch von der einen oder den mehreren Falzlinien eingegrenzt. Bevorzugt weist die Vorrichtung einen Betätigungsbereich in der Mitte der jeweiligen Seitenfläche auf, sodass das Eindrücken des Betätigungsbereichs einerseits für den Nutzer leichter ist und andererseits um die maximale Verformung zu ermöglichen.

Gemäß einer vorteilhaften Ausgestaltung der Erfindung ist vorgesehen, dass der Betätigungsbereich auf einer Seitenfläche des ersten Gehäuseteils vorgesehen ist, welche eine oder mehrere der Durchgangsöffnungen aufweist, durch welche der flüchtige Stoff an die Umgebungsluft abgegeben werden kann. Bei einer derartigen Ausgestaltung ist es möglich, dass sich das Material des Gehäuseteils bei einer Druckeinwirkung durch den Benutzer in die Durchgangsöffnungen hinein bewegen bzw. hinein verformen kann, so dass das Eindrücken des Betätigungsbereichs erleichtert wird. Bevorzugt weisen mehrere, insbesondere zwei diametral gegenüberliegende, Seitenflächen des ersten Gehäuseteils derartige Durchgangsöffnungen und entsprechende Betätigungsbereiche auf.

Gemäß einer vorteilhaften Ausgestaltung ist vorgesehen, dass der Betätigungsbereich an einer Seitenfläche des jeweiligen Gehäuseteils angeordnet ist, an welcher eines der korrespondierenden Rastelemente angeordnet ist. Die Verformung durch die Druckeinwirkung auf den Betätigungsbereich kann bei einer derartigen Ausgestaltung über das Material des Gehäuseteils an das Rastelement weitergeleitet werden, wodurch sich die korrespondierenden Rastelemente der beiden Gehäuseteile voneinander trennen können.

Gemäß einer vorteilhaften Ausgestaltung ist vorgesehen, dass zwei Rastelemente an dem ersten oder zweiten Gehäuseteil an diametral gegenüberliegenden Seitenflächen angeordnet sind, wobei das jeweilige Gehäuseteil zwei durch mindestens eine Falzlinie begrenzte Betätigungsbereiche aufweist, der dazu eingerichtet ist, bei einer Druckeinwirkung durch einen Benutzer der Vorrichtung die korrespondierenden Rastelemente aus dem Rastzustand in einem Freigabezustand zu versetzen, in welchem die beiden Gehäuseteile vollständig voneinander trennbar sind, wobei die Betätigungsbereiche an den Seitenflächen angeordnet sind, welche die Rastelemente aufweisen. Die Verformung durch die Druckeinwirkung auf die Betätigungsbereiche kann bei einer derartigen Ausgestaltung über das Material des Gehäuseteils an das Rastelement weitergeleitet werden, wodurch sich die korrespondierenden Rastelemente der beiden Gehäuseteile voneinander trennen können. Die diametrale Anordnung der Rastelemente und Betätigungsbereiche ermöglicht eine optimale Verteilung der Zugkräfte bei einem Versuch das erste Gehäuseteil und das zweite Gehäuseteil voneinander zu trennen.

Gemäß einer vorteilhaften Ausgestaltung ist vorgesehen, dass die Querschnittsfläche des ersten und des zweiten Gehäuseteils kreisförmig, oval, dreieckig, viereckig oder mehreckig ist. Bei einer runden Querschnittsfläche kann ein Rastelement auf einem definierten Winkelbereich des äußeren Umfangs der runden Querschnittsfläche angeordnet sein. Sofern die Querschnittsfläche des ersten und des zweiten Gehäuseteils rund ist und mindestens zwei Rastelemente auf einem Gehäuseteil angeordnet ist, werden diese Rastelemente bevorzugt diametral auf gegenüberliegenden Seiten des Gehäuseteils angeordnet. Vorteilhafterweise ermöglichen die Kanten, bei einer eckigen Querschnittsfläche, eine einfachere Betätigung des Betätigungsbereichs.

Gemäß einer vorteilhaften Ausgestaltung ist vorgesehen, dass der Betätigungsbereich eine Breite aufweist, die maximal die Hälfte der Breite einer Seite des ersten oder zweiten Gehäuseteils entspricht, bevorzugt maximal ein Drittel der Breite der Seite des ersten oder zweiten Gehäuseteils. Der Betätigungsbereich ist bevorzugt durch die Falzlinien eingegrenzt und kann, bezogen auf die horizontale Achse des stehenden Gehäuses, die Hälfte oder besonders bevorzugt ein Drittel der Breite der Seiten umfassen. Bei einer Ausführung, wobei der Betätigungsbereich ein Drittel der Breite der Seiten umfasst, liegen die Falzlinien am Rastelement näher zusammen und erzeugen eine stärkere Verformung bei Druckeinwirkung als die Ausführung mit einer größeren Breite des Betätigungsbereiches.

Gemäß einer vorteilhaften Ausgestaltung ist vorgesehen, dass das erste oder zweite Gehäuseteil einen Bodenbereich aufweist und das an dem Gehäuseteil angeordnete Rastelement an einer dem Bodenbereich gegenüberliegenden Kante angeordnet ist. Der Bodenbereich des ersten Gehäuseteils beschreibt die unterste Fläche des Gehäuses bei vertikal stehendem Gehäuse. Sofern eine Einlage in dem Gehäuse eingebracht ist, kann diese auf dem Bodenbereich angeordnet werden. Um ein vollständiges Öffnen der Durchlassöffnungen zu ermöglichen, kann das Rastelement am weitest distanzierten Punkt von dem Bodenbereich liegen, sodass das zweite Gehäuseteil und das erste Gehäuseteil die maximale Bauteillänge auseinandergezogen werden können.

Gemäß einer vorteilhaften Ausgestaltung ist vorgesehen, dass sich der Betätigungsbereich ausgehend von dem Bodenbereich des ersten oder zweiten Gehäuseteils in Richtung des Rastelementes verjüngt. Bei einer bevorzugten Ausgestaltungsform mit mindestens zwei Falzlinien können die Falzlinien sich, relativ zur Bodenbereich, vertikal verjüngen, sodass die Falzlinien beim Rastelement einen optimalen Abstand zueinander aufweisen um den Freigabezustand zu erreichen. In einer besonders bevorzugten Ausführungsform beginnen die Falzlinien an den Ecken der unteren Kante, nahe der Bodenbereich, verlaufen in einem 45° Winkel auf die Mitte der Fläche zu und sobald die Breite zwischen den Falzlinien ein Drittel der Breite der Seite aufweist, knicken die beiden Falzlinien vertikal nach oben hin ab.

Gemäß einer vorteilhaften Ausgestaltung ist vorgesehen, dass der Bodenbereich des ersten und/oder des zweiten Gehäuseteils als Automatikboden ausgebildet ist. Der Automatikboden kann eine Mehrzahl von Bodenlaschen aufweisen, welche über Klebelaschen und Klebeflächen derart miteinander verbunden werden, dass das erste und/oder zweite Gehäuseteil mittels eines Handgriffs oder einem maschinellen Fertigungsschritts aufgerichtet werden kann. Vorteilhafterweise kann ein Gehäuseteil mit einem Automatikboden flach zusammengelegt werden und ohne einen Faltschritt wiederaufgerichtet werden. Dies ermöglicht beispielsweise das einfache und platzsparende Verstauen der Gehäuseteile. Darüber hinaus benötigt ein solches Gehäuseteil keine weiteren Verschlussmaterialien, um den Boden zu verschließen.

In einer alternativen bevorzugten Ausgestaltung ist der Bodenbereich des ersten und/oder des zweiten Gehäuseteils als halbautomatischer Boden ausgebildet. Der halbautomatische Boden kann eine Mehrzahl von Bodenlaschen aufweisen, welche derart geformt sind, dass diese zusammengesteckt werden und der Boden verschlossen ist. Ein halbautomatischer Boden benötigt somit keinen Kleber, bzw. keinen Klebeschritt im Fertigungsverfahren. Weiterhin ist es denkbar, dass die Bodenlaschen bei einem halbautomatischen Boden derart ausgebildet sind, dass diese selbstverstärkend wirken. So führt die Befüllung eines solchen Gehäuseteils (und das zusätzliche Gewicht) zur Verstärkung der Verschlusswirkung des Bodens.

Bevorzugt weist das erste Gehäuseteil einen Automatikboden oder einen Halbautomatikboden auf, und das zweite Gehäuseteil weist einen normalen Bodenbereich auf. Der normale Bodenbereich kann im Gehäuse mehr Stabilität erzeugen, wobei dies insbesondere vorteilhaft bei der Betätigung des Druckbereichs ist. Somit kann verhindert werden, dass die Kraft, welche auf den Betätigungsbereich des ersten Gehäuseteils ausgewirkt wird, das zweite Gehäuseteil auch verformt. Ein Automatikboden oder ein Halbautomatikboden kann ferner eine gewisse Beweglichkeit im Gehäuseteil mit dem Betätigungsbereich ermöglichen, wobei ein Freigabezustand der jeweiligen Rastelemente einfacher erreicht wird. Alternativ ist es denkbar, dass das erste oder das zweite Gehäuseteil einen Automatikboden aufweist und das jeweilige andere Gehäuseteil einen Halbautomatikboden aufweist.

In einer weiteren alternativen Ausgestaltung kann das erste Gehäuseteil einen normalen Boden, der insbesondere verklebt oder gesteckt wird, oder einen Automatikboden oder einen Halbautomatikboden aufweisen, wobei korrespondierend dazu das zweite Gehäuseteil entweder die jeweilige gleiche Bodenanordnung oder eine andere Bodenanordnung umfasst.

Gemäß einer vorteilhaften Ausgestaltung ist vorgesehen, dass das Gehäuse einen Innenraum mit einem Volumen im Bereich von 30 ml bis 5 I, insbesondere im Bereich von 30 ml bis 3 I, umfasst. Es ist denkbar, dass in Abhängigkeit des Fassungsvolumens und des zu lagernden Produktes innerhalb des Gehäuses, das Material, die Dicke des Materials und die Qualität des Materials für das erste Material des Gehäuses ausgesucht wird. Vorteilhafterweise kann somit ein stabiles, wiederverwendbares und nachhaltiges Gehäuse für eine Vielzahl an unterschiedlichen Produkten genutzt werden.

Nachfolgend sollen weitere vorteilhafte Ausgestaltungen der Vorrichtung zur Abgabe eines flüchtigen Stoffs erläutert werden.

Bevorzugt ist vorgesehen, dass das erste Material eine Barriereschicht mit einer Barrierefunktion gegen die Permeation des flüchtigen Stoffs aufweist. Unter einer solchen Barriereschicht wird eine Schicht verstanden, die eine geringe Permeabilität für den flüchtigen Stoff aufweist. Durch die Barriereschicht kann verhindert werden, dass der flüchtige Stoff vor einer Aktivierung der Vorrichtung aus dem Gehäuse entweichen kann. Durch die Barriereschicht kann im Inneren des Gehäuses ein derart hoher Partialdruck des flüchtigen Stoffs aufrechterhalten werden, dass ein weiteres Ausdiffundieren des flüchtigen Stoffs aus der Einlage vermieden wird.

Eine vorteilhafte Ausgestaltung der Erfindung sieht vor, dass die Einlage mehrere miteinander durch einen Klebstoff verbundene Lagen des pflanzlichen Faserstoffs aufweist, insbesondere mehrere Lagen Holzschliffpappe. Dadurch, dass die Einlage aus mehreren Lagen des pflanzlichen Faserstoffs gebildet wird, kann das Gewicht der Einlage und damit auch das Gewicht der gesamten Vorrichtung eingestellt werden. Es hat sich herausgestellt, dass die Benutzer derartige Vorrichtungen als wertiger ansehen, wenn die Vorrichtungen ein gewisses Gewicht aufweisen - also nicht zu leicht sind. Daher kann durch die Wahl der Anzahl an Lagen, beispielsweise vier oder fünf oder sechs oder sieben oder acht, ein vorgegebenes Gewicht der Vorrichtung eingestellt werden. Zudem ergibt sich durch das erhöhte Gewicht der Einlage der Vorteil, dass sich der flüchtige Stoff, insbesondere der Duftstoff bzw. der insektizide Wirkstoff, über eine größere Masse der Einlage verteilt. Hierdurch wird der flüchtige Stoff derart verdünnt, dass in der unmittelbaren Nähe des Gehäuses der Vorrichtung kein unangenehm starker Geruch wahrnehmbar ist. Auch dieser Umstand kann dazu beitragen, dass die Vorrichtung von dem Benutzer als wertiger empfunden wird.

Die Einlage kann in das Gehäuse eingelegt und/oder eingeklebt und/oder mittels eines Rückhalteelements festgelegt sein. Das Rückhalteelement kann als Haken, welcher in Aussparungen in dem Gehäuseteilen eingreift ausgebildet sein oder als Vorsprung an einer Innenkontur des Gehäuseteils.

Das erste und das zweite Gehäuseteil haben bevorzugt einen Grundriss von identischer Form, beispielsweise rund, dreieckig, viereckig, insbesondere quadratisch, fünfeckig, sechseckig oder achteckig.

Die Durchgangsöffnungen können hierbei einen runden, ovalen oder polygonalen Querschnitt aufweisen. Durch eine mehrfache Ausführung der Durchgangsöffnung und einer damit einhergehenden erhöhten geöffneten Querschnittsfläche kann mehr und schneller ein flüchtiger Stoff abgegeben werden, wobei somit der gewünschte Effekt des Stoffes schneller endet.

### Kurze Beschreibung der Figuren

Weitere Einzelheiten und Vorteile der Erfindung sollen nachfolgend anhand des in den Zeichnungen dargestellten Ausführungsbeispiels erläutert werden. Hierin zeigt:
- Fig. 1: eine perspektivische Darstellung eines Ausführungsbeispiels eines erfindungsgemäßen Gehäuses in einer Schließstellung;
- Fig. 2: eine perspektivische Darstellung des Gehäuses nach Fig. 1 in einer weiteren Stellung;
- Fig. 3: einen Zuschnitt zur Herstellung eines ersten Gehäuseteils eines Gehäuses nach Fig. 1 und 2;
- Fig. 4: einen Zuschnitt zur Herstellung eines zweiten Gehäuseteils eines Gehäuses nach Fig. 1 und 2;
- Fig. 5: einen Zuschnitt zur Herstellung eines ersten Gehäuseteils eines Gehäuses nach Fig. 1 und 2 mit einem Automatikboden;
- Fig. 6: eine perspektivische Darstellung eines Ausführungsbeispiels einer erfindungsgemäßen Vorrichtung zur Abgabe eines flüchtigen Stoffs an die Umgebungsluft in einer Offenstellung;
- Fig. 7a: eine Draufsicht auf eine Einlage zur Abgabe eines flüchtigen Stoffes;
- Fig. 7b: eine Seitenansicht einer Einlage mit mehreren Schichten;
- Fig. 8: einen Zuschnitt zur Herstellung eines ersten Gehäuseteils einer Vorrichtung nach Fig. 6 und
- Fig. 9: einen alternativen Zuschnitt zur Herstellung eines ersten Gehäuseteils einer Vorrichtung nach Fig. 6 mit einem Automatikboden.

### Ausführungsformen der Erfindung

In den verschiedenen Figuren sind gleiche Teile stets mit den gleichen Bezugszeichen versehen und werden daher in der Regel auch jeweils nur einmal benannt bzw. erwähnt.

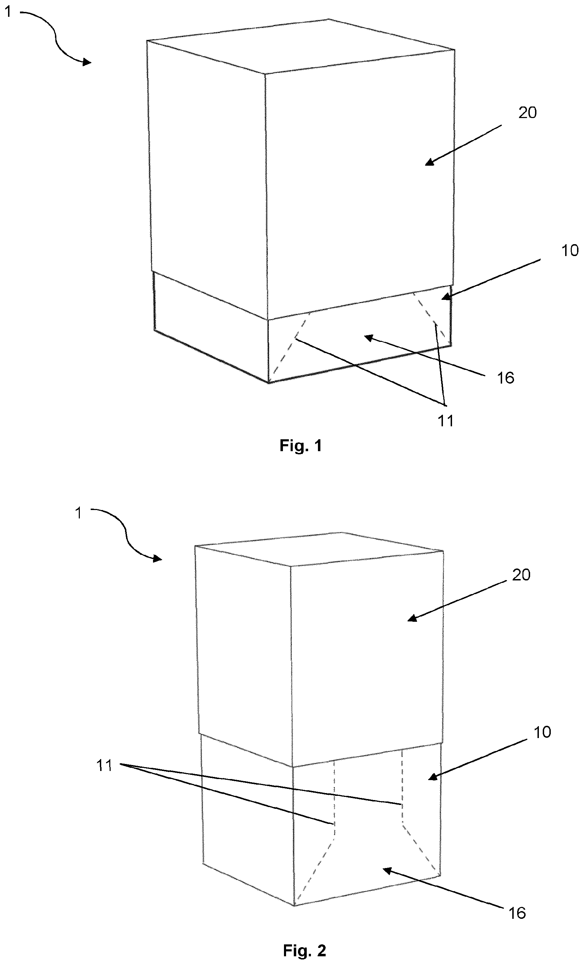

In der Fig. 1 ist ein Ausführungsbeispiel eines erfindungsgemäßen Gehäuses 1 abgebildet. Das Gehäuse 1 besteht aus einem ersten Material, welches einen pflanzlichen Faserstoff aufweist. Das Material kann beispielsweise Papier, Karton, Pappe oder Wellpappe sein. Das Gehäuse 1 umfasst eine erstes Gehäuseteil 10 und ein zweites Gehäuseteil 20, welches eine nach oben weisende Öffnung des ersten Gehäuseteils verschließt. Insofern ist das erste Gehäuseteil 10 in das zweite Gehäuseteil 20 eingeschoben. Die Fig. 1 zeigt eine Schließstellung, in welcher das erste Gehäuseteil 10 maximal in das zweite Gehäuseteil 20 eingefahren ist.

Wie in Fig. 1 erkennbar, weist das erste Gehäuseteil 10 mehrere Falzlinien 11 auf. Eine Falzlinie 11 ist eine durch äußere Einflüsse eingeprägte Linie entlang derer ein gezieltes Knicken des ersten Gehäuseteils 10 ermöglicht werden kann. Die Falzlinien 11 erzeugen einen Betätigungsbereich 16, der von einem Benutzer durch Drücken betätigt werden kann. Erfindungsgemäß können durch eine derartige Druckeinwirkung korrespondiere Rastelemente der Gehäuseteile 10, 20 aus einem Rastzustand in einen Freigabezustand verbracht werden, so dass die Gehäuseteile 10, 20 voneinander getrennt werden können. Ohne die Betätigung des Betätigungsbereichs kann der Rastzustand nicht aufgelöst werden, sodass das vollständige Trennen der beiden Gehäuseteile 10, 20 verhindert wird. Dies ist insbesondere bei der Verwendung des Gehäuses 1 als Verpackung für Reinigungsmittel, Waschparfüm, insbesondere in Form von Pulver, Perlen, Caps oder Pods, oder Waschmitteln vorteilhaft, da schützenswerten Personengruppen somit der Zugang zum Inhalt des Gehäuses 1 erschwert werden kann.

In **Fig. 2** ist die Vorrichtung 1 in einer alternativen Stellung gezeigt, in welcher das zweite Gehäuseteil gegenüber der in Fig. 1 gezeigten Schließstellung nach oben verschoben ist. Es ist erkennbar, dass die Falzlinien 11 des ersten Gehäuseteils 10 ausgehend von einem Bodenbereich 19 des ersten Gehäuseteils 10 aufeinander zulaufen, an einem Knickpunkt abknicken und dann parallel zueinander in einer im Wesentlichen vertikalen Richtung verlaufen. Die Falzlinien 11 erzeugen den Betätigungsbereich 16, welcher ungefähr die Hälfte der Breite der Seitenfläche des ersten oder des zweiten Gehäuseteils 10, 20 aufweisen kann, insbesondere bevorzugt ein Drittel der Breite der Seitenfläche. Der Betätigungsbereich 16 ist im vorliegenden Fall mittig auf einer der Seitenflächen des ersten Gehäuseteils 10 angeordnet.

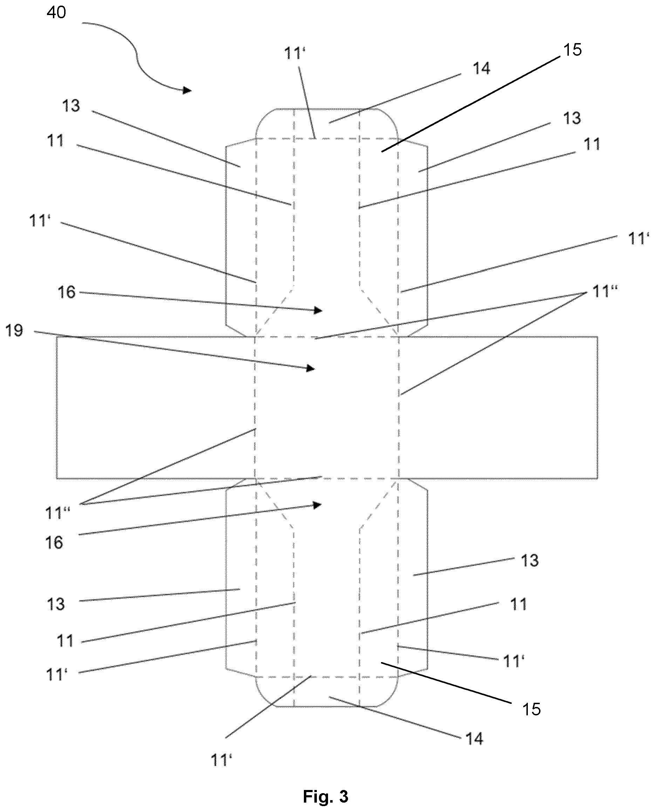

**Fig. 3** zeigt einen ersten Zuschnitt 40 zur Herstellung des ersten Gehäuseteils 10 des Gehäuses 1 aus Fig 1 und 2. Der Zuschnitt weist eine im Wesentlichen kreuzförmige Form auf. Ein Bodenbereich 19 ist über vier senkrecht zueinander angeordnete Falzlinien 11" mit vier Seitenflächen des Gehäuseteils 10 verbunden. Zwei sich diametral gegenüberliegende Seitenflächen 15 der vier Seitenflächen weisen Falzlinien 11 auf. An den dem Bodenbereich 19 gegenüberliegenden Kanten dieser beiden Seitenflächen 15 mit Falzlinien 11 ist jeweils ein Rastelement 14 angeordnet, welches als Lasche ausgestaltet ist, die über eine Falzlinie 11' mit der jeweiligen Seitenfläche 15 verbunden ist. Die Kanten der Seitenflächen 15 mit Falzlinien 11, welchen den Bodenbereich 19 und das Rastelement 14 verbinden sind über eine Falzlinie 11' mit einer Klebelasche 13 verbunden. Die Klebelaschen 13 können beispielsweise mittels eines Klebstoffs an eine benachbarte Seitenfläche des ersten Gehäuseteils 10 adhäsiv angebracht werden. Dazu werden die Klebelaschen 13 vorher nach innen, um die Falzlinie 11', geknickt

Die Seitenflächen 15, welche die Falzlinien 11 aufweisen, umfassen zudem einen durch zwei Falzlinien 11 begrenzten Betätigungsbereich 16, der dazu eingerichtet ist bei einer Druckeinwirkung durch einen Benutzer der Vorrichtung die korrespondierenden Rastelemente 14 aus dem Rastzustand in einem Freigabezustand zu versetzen, in welchem die beiden Gehäuseteile 10, 20 des Gehäuses 1 vollständig voneinander trennbar sind. Eine Falzlinie 11 einer Seitenfläche 15 kann sich auch über das an der Seitenfläche 15 angeordnete Rastelement 14 erstrecken. Dies verstärkt bzw. vereinfacht die Verformung des Rastelements 14 bei der Betätigung des Betätigungsbereichs 16 und somit wird der Übergang vom Rastzustand zum Freigabezustand vereinfacht.

Bevorzugt besteht das an dem ersten Gehäuseteil 10 vorhandene Rastelemente 14 aus dem jeweiligen Material des ersten Gehäuseteils 10, wobei das Rastelement 14 ein an dem jeweiligen ersten Gehäuseteil 10 umlegbarer Abschnitt ist. Ferner ist das Rastelement 14 bevorzugt an einer dem Bodenbereich 19 gegenüberliegenden Kante angeordnet.

Eine Falzlinie 11 kann in dem ersten und/oder zweiten Gehäuseteil 10, 20 als Falzung, Nutung, Rillung oder Perforation ausgebildet sein. Ein Materialabtrag oder eine Krafteinwirkung auf den Zuschnitt ermöglichen ein einfaches und kontrolliertes knicken, um die vordefinierte Falzlinie 11, 11', 11".

Die Falzlinien 11 des ersten Gehäuseteils sind bevorzugt derart ausgestaltet, dass sich der Betätigungsbereich 16 ausgehend von dem Bodenbereich 19 des ersten oder zweiten Gehäuseteils 10, 20 in Richtung des Rastelementes 14, 24 verjüngt.

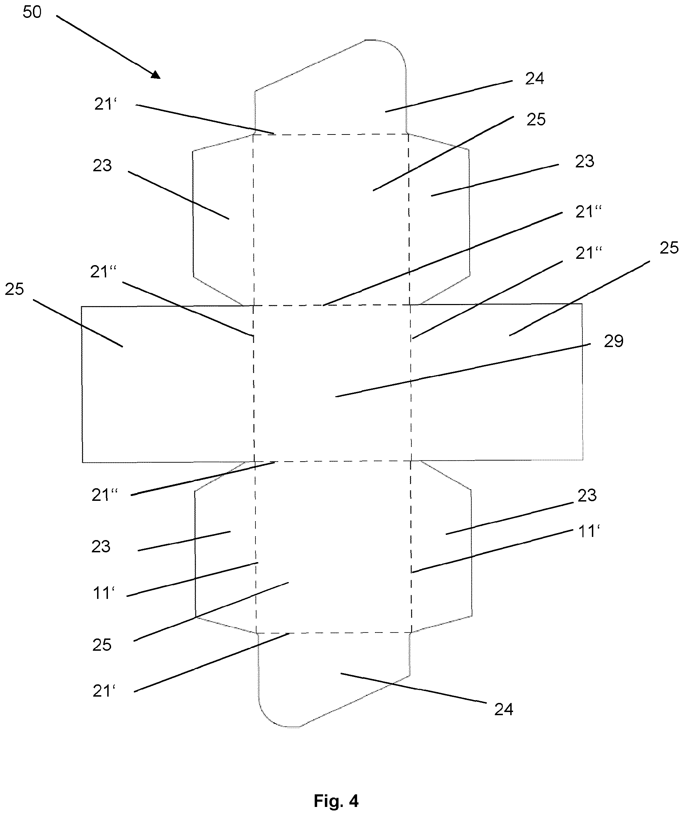

In **Fig. 4** ist ein zweiter Zuschnitt 50 dargestellt, aus dem das zum ersten Gehäuseteil 10 korrespondierende zweite Gehäuseteil 20 gemäß Fig. 1 und 2 geformt werden kann. Der zweite Zuschnitt 50 ist ebenfalls im Wesentlichen kreuzförmig ausgestaltet und weist eine Deckelfläche 29 auf, die über vier senkrecht zueinander angeordnete Falzlinien 11" mit vier Seitenflächen 25 des zweiten Gehäuseteils 20 verbunden ist. Zwei sich diametral gegenüberliegende Seitenflächen der vier Seitenflächen 25 weisen Rastelemente 24 auf. Diese Rastelemente 24 sind ebenfalls als umlegbare Abschnitt bzw. Laschen ausgestaltet und bestehen bevorzugt aus dem jeweiligen Material des zweiten Gehäuseteils 20.

Die zweiten Rastelemente 24 des zweiten Gehäuseteils 20 können mit den ersten Rastelemente 14 des ersten Gehäuseteils verrasten und somit ein unbeabsichtigtes Lösen der Verbindung des ersten und zweiten Gehäuseteils 10, 20 voneinander verhindern. Das zweite Rastelement 24 kann nach dem umlegen relativ zu der Seitenfläche optional an der Innenseite der jeweiligen Seitenfläche angeklebt werden.

Ferner sind die Rastelemente 24 bevorzugt an einer der Deckelfläche gegenüberliegenden Kante angeordnet.

Zur Bildung des Gehäuses 1 werden bevorzugt zuerst aus dem ersten und dem zweiten Zuschnitt aus Fig. 3 und Fig. 4 zunächst das erste und das zweite Gehäuseteil 10, 20 erzeugt. Zudem werden die Rastelemente 14, 24, durch umlegen der entsprechenden Abschnitte bzw. Laschen nach außen, derart nutzbar gemacht, dass ein Rastzustand beim Einschieben des ersten Gehäuseteils 10 in das zweite Gehäuseteil 20 erreicht werden kann. Die korrespondierenden Rastelemente 14, 24 sind bevorzugt am ersten und zweiten Gehäuseteil 10, 20 bzw. Zuschnitt 40, 50 auf den korrespondieren Seitenflächen angebracht.

Beim Einschieben des ersten Gehäuseteils 10 in das zweite Gehäuseteil 20 können das erste und das zweite Rastelement 14, 24 zwischen einer äußeren Seitenfläche des zweiten Gehäuseteils 20 und einer korrespondierenden, äußeren Seitenfläche des ersten Gehäuseteils 10 eingeklemmt werden. Das erste Rastelement 14 ist hin zum Bodenbereich 19 abgeknickt und das zweite Rastelement 24 ist weg vom Bodenbereich 19 geknickt. Die ineinandergreifenden ersten und zweiten Rastelemente 14, 24 können ein vollständiges Trennen des ersten Gehäuseteils 10 vom zweiten Gehäuseteil 20 verhindern.

Um einen Freigabezustand zu erreichen, in welchem sich die beiden Gehäuseteile 10, 20 trennen lassen, kann ein Betätigungsbereich 16 oder können beide Betätigungsbereiche 16 an dem ersten Gehäuseteil 10, nach innen gedrückt werden. Diese Druckeinwirkung kann eine Verformung der gesamten Seitenfläche 15 und des ersten Rastelementes 14 erzeugen, wodurch die beiden Rastelemente 14, 24 voneinander getrennt werden.

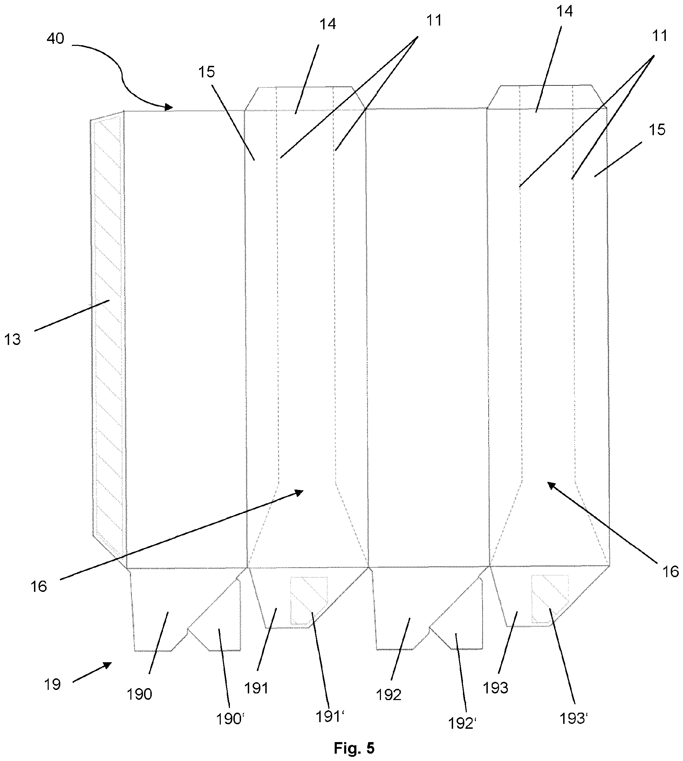

**Fig. 5** zeigt einen alternativen Zuschnitt 40 für ein erstes Gehäuseteil 10. Dieser Zuschnitt 40 weist einen Automatikboden auf, wobei der Bodenbereich wo19 mehrere Bodenlaschen 190, 191, 192, 193, Klebelaschen 190', 192' und Klebeflächen 191', 193' umfasst. Diese Klebelaschen 190', 192` werden bevorzugt derart mit den Klebeflächen 191', 193` verbunden, dass das erste Gehäuseteil 10 mit einem Handgriff bzw. einem Aufstellschritt aufgerichtet werden kann. In einem ersten Schritt wird die Klebelasche 13 bevorzugt auf der entferntesten Seitenfläche angebracht, sodass die Kante zwischen der Klebelasche 13 und der naheliegenden Seitenfläche auf der entferntesten Kante des Zuschnitts liegt. Folgend auf den ersten Schritt oder parallel zum ersten Schritt wird bevorzugt die Klebelasche 190` auf der Klebefläche 191' und die Klebelasche 192` auf der Klebefläche 193` angebracht. Die daraus entstehenden Flächen können vorteilhafterweise derart zusammengesteckt werden, dass das erste Gehäuseteil 10 mit einem Handgriff aufgerichtet werden kann und die Bodenfläche 19 verschlossen ist. Alternativ kann das erste Gehäuseteil 10 einen Halbautomatikboden aufweisen, wobei dieser keinen Klebeschritt benötigt. Hierbei werden die vier Bodenlaschen 190, 191, 192, 193 derart zusammengesteckt, dass die Bodenfläche 19 verschlossen ist. Bevorzugt kann ein Halbautomatikboden auch selbstverstärkend ausgestaltet sein, wobei die Bodenlaschen derart überlappend ausgestaltet sind, dass bei der Aufbringung einer Kraft auf die Bodenfläche 19 die Bodenfläche 19 fester verschließt.

Ferner weist die Ausgestaltung aus Fig. 5 mehrere Falzlinien 11 auf, welche einen Betätigungsbereich 16 erzeugen.

Das erste und das zweite Gehäuseteil 10, 20 weisen bevorzugt einen kreisförmigen, ovalen, dreieckigen, viereckigen oder mehreckigen Querschnitt auf, wobei insbesondere bevorzugt die Querschnittsflächen des ersten und des zweiten Gehäuseteils 10, 20 derart korrespondieren, dass das erste Gehäuseteil 10 in das zweite Gehäuseteil 20 eingeschoben werden kann und die jeweils korrespondieren Rastelemente 14, 24 miteinander verrasten können.

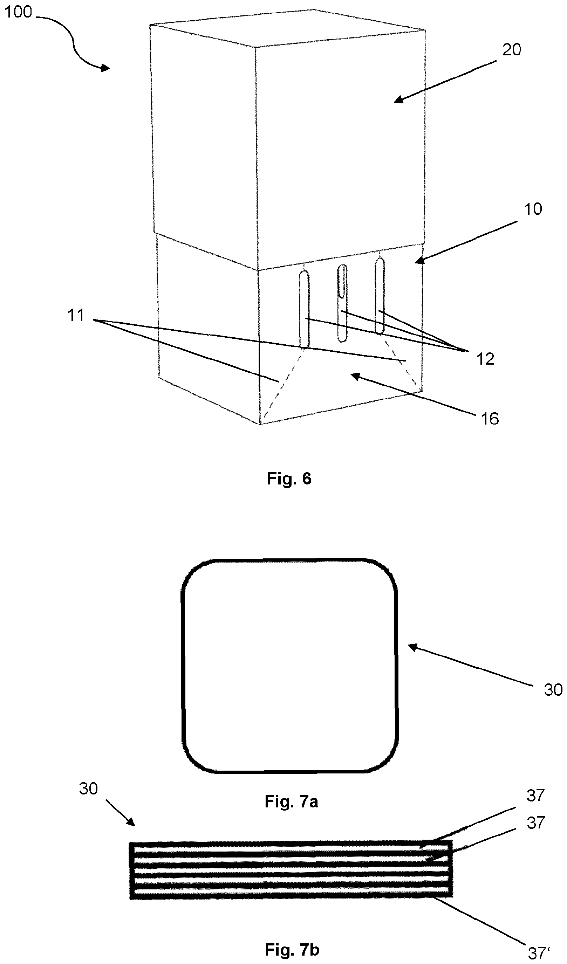

Nachfolgend wird anhand der **Fig. 6 bis 8** ein Ausführungsbeispiel einer erfindungsgemäßen Vorrichtung 100 zur Abgabe eines flüchtigen Stoffs, insbesondere eines Duftstoffs oder eines insektiziden Wirkstoffes, an die Umgebungsluft, erläutert. Die Vorrichtung 100 umfasst ein Gehäuse 1 welches im Wesentlichen dem in Fig. 1 und 2 gezeigten Gehäuse 1 entspricht. Im Unterschied zu dem Gehäuse 1 nach Fig. 1 und 2 umfasst das Gehäuse 1 der Vorrichtung 1 ein erstes Gehäuseteil 10 mit mehreren in Fig. 1 nicht sichtbaren Durchgangsöffnungen 12 und ein zweites Gehäuseteil 20, welches die Durchgangsöffnungen in der in Fig. 1 gezeigten Schließstellung verdeckt. Insofern ist das erste Gehäuseteil 10 in das zweite Gehäuseteil 20 eingeschoben.

Innerhalb des Gehäuses 1 ist eine Einlage 30 angeordnet, welche den Duft- oder Wirkstoff aufweist und diesen an die Umgebungsluft abgibt. Das Material der Einlage 30 ist so ausgewählt, dass dieses den flüchtigen Stoff in einer auf den Nutzen angepassten Zeitdauer abgeben kann. Die Einlage 30 besteht bevorzugt aus einem pflanzlichen Faserstoff, insbesondere Papier, Karton oder Pappe, wobei das Material mit dem flüchtigen Stoff versehen, insbesondere imprägniert, ist.

Wie in Fig. 1 erkennbar, weist das erste Gehäuseteil 10 in mehrere Falzlinien 11 auf. Eine Falzlinie 11 ist eine durch äußere Einflüsse in das Gehäuse 1 eingeprägte Linie entlang derer ein gezieltes Knicken des Gehäuses 1 ermöglicht werden kann. Die Falzlinien 11 erzeugen einen Betätigungsbereich 16, der von einem Benutzer durch Drücken betätigt werden kann. Erfindungsgemäß können durch eine derartige Druckeinwirkung korrespondiere Rastelemente der Gehäuseteile 10, 20 aus einem Rastzustand in einen Freigabezustand verbracht werden, so dass die Gehäuseteile voneinander getrennt werden können.

In **Fig. 6** ist die Vorrichtung 1 in einer Offenstellung gezeigt in welcher die Durchgangsöffnungen des ersten Gehäuseteils zur Abgabe des flüchtigen Stoffs freigegeben sind. Es ist erkennbar, dass die Falzlinien 11 an den Seitenflächen des ersten Gehäuseteils 10 angeordnet sind, welche die Durchgangsöffnungen 12 aufweisen. Die Falzlinien 11 verlaufen ausgehend von einem Bodenbereich des ersten Gehäuseteils aufeinander zulaufend, knicken an einem Knickpunkt ab und verlaufen dann parallel zueinander in einer im Wesentlichen vertikalen Richtung. Die Falzlinien 11 erzeugen den Betätigungsbereich 16, welcher ungefähr die Hälfte der Breite der Seitenfläche aufweisen kann, insbesondere bevorzugt ein Drittel der Breite der Seitenfläche. Der Betätigungsbereich 16 ist im vorliegenden Fall mittig auf der jeweiligen Seitenfläche des ersten Gehäuseteils 10 angeordnet.

**Fig. 7a und 7b** zeigen eine Einlage 30 die bei dem Ausführungsbeispiel gemäß Fig. 6Verwendung finden kann. Diese Einlage 30 weist einen quadratischen Querschnitt mit abgerundeten Ecken auf. Die Einlage 20 besteht aus mehreren, hier fünf, miteinander durch einen Klebstoff verbundenen Lagen 37 des pflanzlichen Faserstoffs, beispielsweise Holzschliffpappe. Der Klebstoff ist bevorzugt biologisch abbaubar ist. Die Lagen weisen jeweils ein Flächengewicht im Bereich von 350 g/m² bis 950 g/m², bevorzugt von 450 g/m² bis 600 g/m² oder von 600 g/m² bis 800 g/m², auf. Alternativ kann vorgesehen sein, dass die Lagen jeweils eine Dicke im Bereich von 0,8 mm bis 2,5 mm und ein Flächengewicht im Bereich von 350 g/m² bis 950 g/m² aufweisen.

Alternativ kann die Form der Einlage kreisförmig, oval oder polygonal sein kann. Eine dieser mehreren Lagen 37 stellt eine Bodenlage 37` dar. Die Bodenlage 37` weist keine Öffnungen auf und ist die Lage, welche zu einer nächstgelegenen Auflage- oder Befestigungsfläche positioniert ist. Lediglich alle weiteren Lagen 37 können Öffnungen mit beliebigen Formen aufweisen. Über den Querschnitt der Öffnungen kann die Oberfläche zur Abgabe des flüchtigen Stoffes der Einlage 30 angepasst werden.

Die Einlage 30 kann einerseits in dem ersten Gehäuseteil 10 oder in dem zweiten Gehäuseteil 20 platziert werden. In einer besonders bevorzugten Ausführungsform wird die Einlage in das erste Gehäuseteil 10 auf einen Bodenbereich 19 gelegt oder geklebt oder über Vorrichtungen befestigt. Alternativ kann die Einlage 30 in das zweite Gehäuseteil 20 eingeklebt werden oder über mögliche Vorrichtungen an einer Stelle angeordnet werden.

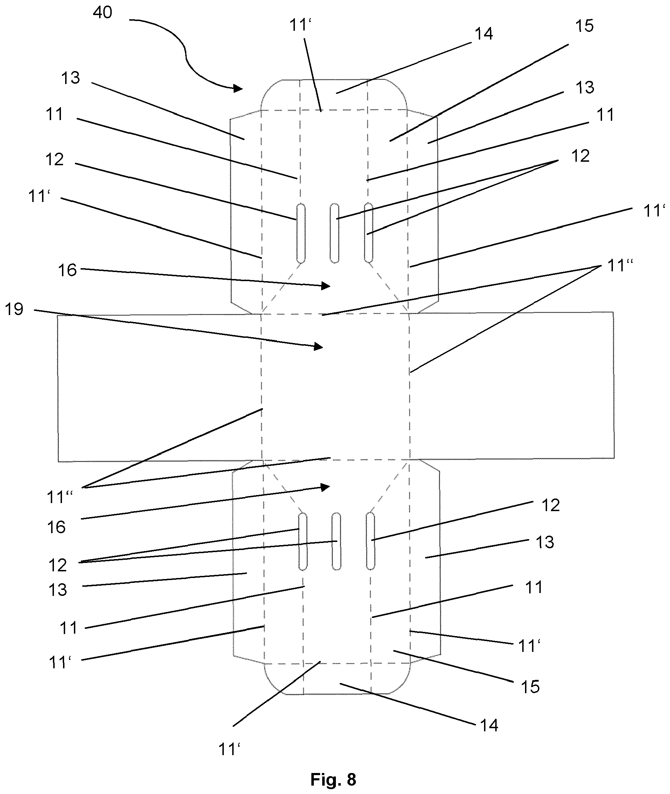

**Fig. 8** zeigt einen Zuschnitt 100 zur Herstellung des ersten Gehäuseteils 10 des Gehäuses 1 aus Fig 6. Der Zuschnitt 100 weist eine im Wesentlichen kreuzförmige Form auf. Ein Bodenbereich 19 ist über vier senkrecht zueinander angeordnete Falzlinien 11" mit vier Seitenflächen des Gehäuseteils verbunden. Zwei sich diametral gegenüberliegende Seitenflächen 15 der vier Seitenflächen weisen Durchgangsöffnungen 12 auf. An den dem Bodenbereich 19 gegenüberliegenden Kanten dieser beiden Seitenflächen 15 mit Durchgangsöffnungen 12 ist jeweils ein Rastelement 14 angeordnet, welches als Lasche ausgestaltet ist, die über eine Falzlinie 11' mit der jeweiligen Seitenfläche verbunden ist. Die Kanten der Seitenflächen 15 mit Durchgangsöffnungen 12, welchen den Bodenbereich 19 und das Rastelement 14 verbinden sind über eine Falzlinie 11' mit einer Klebelasche 13 verbunden. Die Klebelaschen 13 können beispielsweise mittels eines Klebstoffs an eine benachbarte Seitenfläche des ersten Gehäuseteils 10 adhäsiv angebracht werden. Dazu werden die Klebelaschen 13 vorher nach innen, um die Falzlinie 11', geknickt.

Die Seitenflächen 15, welche die Durchgangsöffnungen 12 aufweisen, umfassen zudem einen durch zwei Falzlinien 11 begrenzten Betätigungsbereich 16, der dazu eingerichtet ist, bei einer Druckeinwirkung durch einen Benutzer der Vorrichtung die korrespondierenden Rastelemente 14 aus dem Rastzustand in einem Freigabezustand zu versetzen, in welchem die beiden Gehäuseteile 10, 20 des Gehäuses 1 vollständig voneinander trennbar sind.

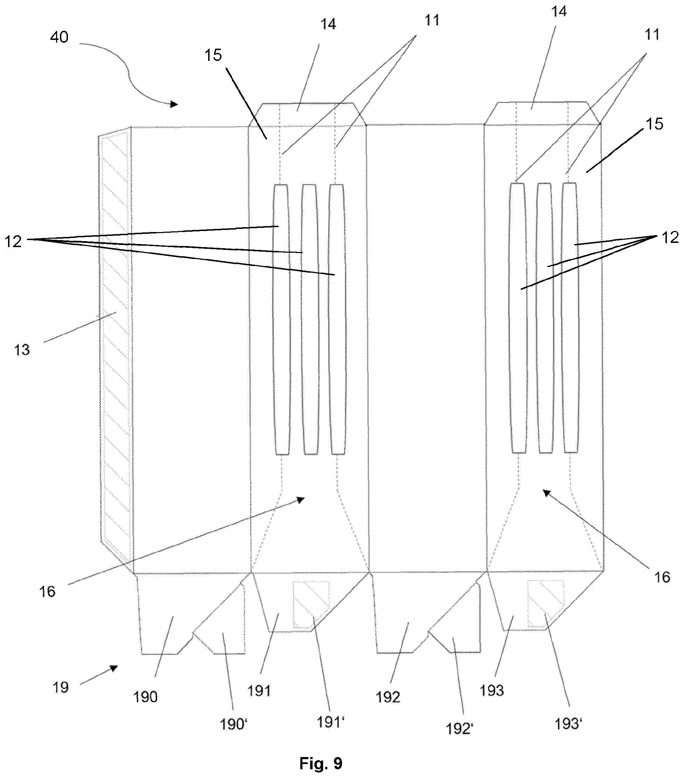

**Fig. 9** zeigt einen Zuschnitt für ein erstes Gehäuseteil 10, ähnlich dem aus Fig. 5, mit mehreren Durchgangsöffnungen 12.

Bei den alternativen Zuschnitten 40, 50 für die Gehäuseteile 10, 20 können dieselben Vorteile und Wirkungen erreicht werden, die bereits im Zusammenhang mit dem erfindungsgemäßen Gehäuse 1 oder den Zuschnitten 40, 50 oder der Vorrichtung zur Abgabe eines flüchtigen Stoffs 100 beschrieben worden sind. Die im Zusammenhang mit dem erfindungsgemä-ßen Gehäuse 1 oder den Zuschnitten 40, 50 oder der Vorrichtung zur Abgabe eines flüchtigen Stoffs 100 beschriebenen vorteilhaften Ausgestaltungen und Merkmale können auch bei den alternativen Zuschnitten 40, 50, allein oder in Kombination, Anwendung finden.

Die vorstehend beschriebene Vorrichtung 100 zur Abgabe eines flüchtigen Stoffs, insbesondere eines Duftstoffs oder eines insektiziden Wirkstoffes, an die Umgebungsluft, umfasst ein Gehäuse 1 aus einem ersten Material, welches einen pflanzlichen Faserstoff, insbesondere ein Papier, einen Karton oder eine Pappe, aufweist und eine innerhalb des Gehäuses angeordneten Einlage 30 aus einem zweiten Material, die mit dem flüchtigen Stoff versehen ist, wobei das Gehäuse 1 ein erstes Gehäuseteil 10 und ein zweites Gehäuseteil 20 umfasst, wobei das erste Gehäuseteil 10 und/oder das zweite Gehäuseteil 20 eine Aufnahme für die Einlage 30 umfasst, wobei das erste Gehäuseteil 10 eine oder mehrere Durchgangsöffnungen 12 aufweist, durch welche der flüchtige Stoff in die Umgebungsluft abgegeben werden kann, und das zweite Gehäuseteil 20 aus einer Schließstellung, in welcher es die Durchgangsöffnungen 12 des ersten Gehäuseteils 10 verdeckt, in eine Offenstellung verschiebbar ist, in welcher die Durchgangsöffnungen 12 des ersten Gehäuseteils 10 zur Abgabe des flüchtigen Stoffs freigegeben sind, wobei an dem ersten und an dem zweiten Gehäuseteil 10, 20 korrespondierende Rastelemente 14, 24 angeordnet ist, die in einem Rastzustand ein vollständiges Trennen der bei-den Gehäuseteile 10, 20 voneinander verhindern, wobei das erste Gehäuseteil 10 und/oder das zweite Gehäuseteil 20 einen durch mindestens eine Falzlinie 11 begrenzten Betätigungsbereich 16 aufweist, der dazu eingerichtet ist, bei einer Druckeinwirkung durch einen Benutzer der Vorrichtung die korrespondieren-den Rastelemente 14, 24 aus dem Rastzustand in einem Freigabezustand zu versetzen, in welchem die beiden Gehäuseteile 10, 20 vollständig voneinander trennbar sind. Hierdurch kann die Wiederverwendbarkeit des Gehäuses 1 verbessert werden.

### Bezugszeichenliste

- 1: Gehäuse
- 10: erstes Gehäuseteil
- 11: Falzlinie (Betätigungsbereich)
- 11': Falzlinie (zwischen Seitenfläche und Klebelasche bzw. Rastelement
- 11": Falzlinie (zwischen Knickbereich Bodenbereich und Seitenflächen)
- 12: Durchgangsöffnungen
- 13: Klebelasche
- 14: erstes Rastelement
- 15: Seitenfläche
- 16: Betätigungsbereich
- 19: Bodenbereich
- 20: zweites Gehäuseteil
- 21': Falzlinie (zwischen Seitenfläche und Klebelasche bzw. Rastelement
- 21": Falzlinie (zwischen Knickbereich Bodenbereich und Seitenflächen)
- 23: Klebelasche
- 24: zweites Rastelement
- 25: Seitenfläche
- 29: Deckelfläche
- 30: Einlage
- 37: Lage
- 37': Bodenlage
- 40: Zuschnitt für ein erstes Gehäuseteil
- 50: Zuschnitt für ein zweites Gehäuseteil
- 100: Vorrichtung zur Abgabe eines flüchtigen Stoffs
- 190, 191, 192, 193: Bodenlaschen
- 190', 192`: Klebelasche
- 191', 193`: Klebefläche

## Patentansprüche

1. Gehäuse (1) aus einem ersten Material, welches einen pflanzlichen Faserstoff, insbesondere ein Papier, einen Karton oder eine Pappe, aufweist
wobei das Gehäuse (1) ein erstes Gehäuseteil (10) und ein zweites Gehäuseteil (20) umfasst,
wobei an dem ersten und an dem zweiten Gehäuseteil (10, 20) korrespondierende Rastelemente (14, 24) angeordnet ist, die in einem Rastzustand ein vollständiges Trennen der beiden Gehäuseteile (10, 20) voneinander verhindern,
**dadurch gekennzeichnet, dass**
das erste Gehäuseteil (10) und/oder das zweite Gehäuseteil (20) einen durch mindestens eine Falzlinie (11) begrenzten Betätigungsbereich (16) aufweist, der dazu eingerichtet ist, bei einer Druckeinwirkung durch einen Benutzer der Vorrichtung die korrespondierenden Rastelemente (14, 24) aus dem Rastzustand in einem Freigabezustand zu versetzen, in welchem die beiden Gehäuseteile (10, 20) vollständig voneinander trennbar sind.

2. Gehäuse (1) nach Anspruch 1, **dadurch gekennzeichnet, dass** die mindestens eine Falzlinie (11) in dem ersten und/oder zweiten Gehäuseteil (10, 20) als Falzung oder Nutung oder Rillung oder Perforation ausgebildet ist.

3. Gehäuse (1) nach einem der vorhergehenden Ansprüche, **dadurch gekennzeichnet, dass** die an einem ersten und einem zweiten Gehäuseteil (10, 20) vorhandenen Rastelemente (14, 24) aus dem jeweiligen Material des ersten oder des zweiten Gehäuseteils (10, 20) bestehen, wobei das Rastelement (14, 24) ein an dem jeweiligen ersten oder zweiten Gehäuseteil (10, 20) umlegbarer Abschnitt ist.

4. Gehäuse (1) nach einem der vorhergehenden Ansprüche, **dadurch gekennzeichnet, dass** der Betätigungsbereich (16) durch mehrere Falzlinien (11) begrenzt ist.

5. Gehäuse (1) nach einem der vorhergehenden Ansprüche, **dadurch gekennzeichnet, dass** der Betätigungsbereich (19) durch zwei parallel verlaufend angeordnete Falzlinien (11) begrenzt ist.

6. Gehäuse (1) nach einem der vorhergehenden Ansprüche, **dadurch gekennzeichnet, dass** das erste Gehäuseteil (10) und/oder das zweite Gehäuseteil (20) mindestens einen Betätigungsbereich (16) aufweist, wobei dieser mittig auf einer Seitenfläche (15) des Gehäuseteils (10) angeordnet ist.

7. Gehäuse (1) nach einem der vorhergehenden Ansprüche, **dadurch gekennzeichnet, dass** der Betätigungsbereich (16) an einer Seitenfläche (15) des jeweiligen Gehäuseteils (10, 20) angeordnet ist, an welcher eines der korrespondierenden Rastelemente (14) angeordnet ist.

8. Gehäuse (1) nach einem der vorhergehenden Ansprüche, **dadurch gekennzeichnet, dass** zwei Rastelemente (14) an dem ersten oder zweiten Gehäuseteil (10, 20) an diametral gegenüberliegenden Seitenflächen (15) angeordnet sind, wobei das jeweilige Gehäuseteil (10, 20) zwei durch mindestens eine Falzlinie (11) begrenzte Betätigungsbereiche (16) aufweist, der dazu eingerichtet ist, bei einer Druckeinwirkung durch einen Benutzer der Vorrichtung die korrespondierenden Rastelemente (14) aus dem Rastzustand in einem Freigabezustand zu versetzen, in welchem die beiden Gehäuseteile (10, 20) vollständig voneinander trennbar sind, wobei die Betätigungsbereiche (16) an den Seitenflächen (15) angeordnet sind, welche die Rastelemente (14) aufweisen.

9. Gehäuse (1) nach einem der vorhergehenden Ansprüche, **dadurch gekennzeichnet, dass** eine Querschnittsfläche des ersten und des zweiten Gehäuseteils (10, 20) kreisförmig, oval, dreieckig, viereckig oder mehreckig ist

10. Gehäuse (1) nach einem der vorhergehenden Ansprüche, **dadurch gekennzeichnet, dass** der Betätigungsbereich (16) eine Breite aufweist, die maximal die Hälfte der Breite einer Seite des ersten oder zweiten Gehäuseteils (10, 20) entspricht, bevorzugt maximal ein Drittel der Breite der Seite des ersten oder zweiten Gehäuseteils (10, 20).

11. Gehäuse (1) nach einem der vorhergehenden Ansprüche, **dadurch gekennzeichnet, dass** das erste oder zweite Gehäuseteil (10, 20) einen Bodenbereich (19) aufweist und das an dem Gehäuseteil (10, 20) angeordnete Rastelement (14) an einer dem Bodenbereich (19) gegenüberliegenden Kante angeordnet ist.

12. Gehäuse (1) nach Anspruch 11, **dadurch gekennzeichnet, dass** sich der Betätigungsbereich (16) ausgehend von dem Bodenbereich (19) des ersten oder zweiten Gehäuseteils (10) in Richtung des Rastelementes (14) verjüngt.

13. Gehäuse (1) nach einem der Ansprüche 11 oder 12, **dadurch gekennzeichnet, dass** der Bodenbereich (19) des ersten und/oder des zweiten Gehäuseteils (10, 20) als Automatikboden ausgebildet ist.

14. Vorrichtung zur Abgabe eines flüchtigen Stoffs, insbesondere eines Duftstoffs oder eines insektiziden Wirkstoffes, an die Umgebungsluft,
mit einem Gehäuse (1) nach einem der vorhergehenden Ansprüche und
mit einer innerhalb des Gehäuses (1) angeordneten Einlage (30) aus einem zweiten Material, die mit dem flüchtigen Stoff versehen ist,
wobei das erste Gehäuseteil (10) und/oder das zweite Gehäuseteil (20) eine Aufnahme für die Einlage (30) umfasst,
wobei das erste Gehäuseteil (10) eine oder mehrere Durchgangsöffnungen (12) aufweist, durch welche der flüchtige Stoff in die Umgebungsluft abgegeben werden kann, und das zweite Gehäuseteil (20) aus einer Schließstellung, in welcher es die Durchgangsöffnungen (12) des ersten Gehäuseteils (10) verdeckt, in eine Offenstellung verschiebbar ist, in welcher die Durchgangsöffnungen (12) des ersten Gehäuseteils (10) zur Abgabe des flüchtigen Stoffs freigegeben sind.

15. Vorrichtung nach einem der vorhergehenden Ansprüche, **dadurch gekennzeichnet, dass** der Betätigungsbereich (16) auf einer Seitenfläche (15) des ersten Gehäuseteils (10) vorgesehen ist, welche eine oder mehrere der Durchgangsöffnungen (12) aufweist, durch welche der flüchtige Stoff an die Umgebungsluft abgegeben werden kann.
